# EUROPEAN PATENT SPECIFICATION

(11) **EP 4 049 639 B1**
(45) Date of publication and mention of the grant of the patent: **31.01.2024**
(21) Application number: 21210120.8
(22) Date of filing: 10.04.2018
(51) Int. Cl.: A61F 13/02

(54) **WOUND DRESSING**
WUNDVERBAND
PANSEMENT DE PLAIE

(30) Priority: 11.04.2017 GB 201705800; 04.07.2017 GB 201710771
(43) Date of publication of application: 31.08.2022
(62) Divisional of application: 18725892.6
(73) Proprietor: Pellis Innovations Limited, Edinburgh EH3 8PB (GB)
(72) Inventor: McCormack, Brian, Fife, KY2 6QS (GB)
(74) Representative: Myint, Julie Marie

(56) References cited:
- GB-A- 1 379 158
- US-A1- 2016 045 634

## Description

The present invention relates to a bandage.

Wound dressings, surgical tape, bandages and plasters are widely used to cover wounds, hold together wounds or cover simple cuts. Previously proposed wound dressings, surgical tape, bandages and plasters have used sticky fabric, adhesive plastics film and suchlike to attach them to a patient's skin. In the case of a wound dressing or plaster the sticky fabric, adhesive plastic film holds an absorbent pad, often impregnated with anti-septic or other topical medicine, on the wound or skin of the patient. Such pads are used to absorb leaking fluid or protect against foreign bodies entering the wound and necessarily the adhesion is designed to hold the pad securely against the skin. It is normally necessary to replace such plasters or dressings regularly due to leakage of fluid from the wound with a fresh dressing or a different type of dressing. The replacement of such a wound dressing or plaster involves tugging off the old plaster or dressing thereby often causing further damage to the wound. In the case of surgical tape for instance is often used in operations to hold people's eyes shut during an operation and removing the tape particularly from elderly patients causes distress.

A cannula is a hollow tube with a sharp, retractable inner core that can be inserted into a vein, an artery, or another body cavity. It is generally covered with a specific dressing to hold it in place and stop the patient accidentally disturbing it.

When removing a traditional cannula dressing, there can be a pain and discomfort due to the skin being pulled whilst the cannula is still in place in the vein.

It is an aim of the present invention to provide a bandage which seeks to overcome these previously mentioned disadvantages.

Accordingly the present invention is directed to a bandage of elongate form in which sections of the bandage can be of a woven or non-woven fabric, comprising multiple sections, wherein the sections are held together via water-soluble fibre material. This provides the advantage that if a wound dressing is covered by a standard elongate bandage the bandage can be removed carefully particularly when soiled in sections.

In a preferred embodiment the sections are sewn together with water soluble thread. Advantageously the sections are joined together by non-woven soluble material such as paper fibres which are joined with either each section of the bandage by needling or alternatively adhesive. This provides the advantage of a long bandage being produced with multiple sections which can easily be broken apart by water. In the case of the sections of soluble material this gives a significant break between the sections of bandage.

Examples of the present invention will now be described hereinbelow with reference to the accompanying drawings, in which:

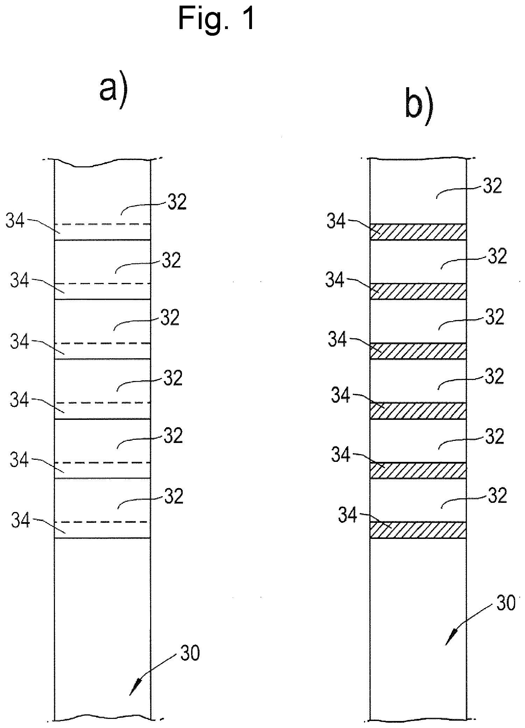

Figure 1 shows top views of bandages made in accordance with the present invention.

Figure 1 shows to plan views of an elongate bandage 30. Each bandage 30 is comprised of a number of sections 32. The bandage 30 can be of a woven cloth fabric or alternatively of a non-woven fabric. The size of each section 32 of the bandage 30 can be varied and will be dependent upon the intended application. In Figure 1a the sections 32 are sewn together with soluble thread at points 34. In Figure 1b there exists a section of soluble material 34 which is joined to each section of bandage 32. The joint between sections here can be achieved nonwoven fashion by needling or alternatively by adhesive or other fixing means. The soluble material may be soluble paper or non-woven soluble paper/wood fibre.

Water is applied to the bandage 30 breaking the sections 32 and thus allowing careful removal of each section 32 to occur. Obviously the bandage of Figure 1b allows discrete breaks in the bandage 30 and may be suitable for certain purposes where the breakup of the elongate bandage 30 is more required.

## Claims

1. A bandage (30) of elongate form **characterised in that** it is made of sections (32) of bandage of a woven or non-woven fabric, comprising multiple sections (32), wherein the sections are held together (34) via water-soluble fibre material.

2. A bandage of elongate form according to claim 1 in which the sections of the bandage are sewn together with water-soluble thread.

3. A bandage of elongate form according to claim 1, in which the sections of the bandage (32) are joined together by non-woven water-soluble material which are joined with each section of the bandage by needling.

4. A bandage of elongate form according to claim 1, in which the sections of the bandage (32) are joined together by non-woven water-soluble material which are joined with each section of the bandage by adhesive.

## Patentansprüche

1. Bandage (30) mit länglicher Form, **dadurch gekennzeichnet, dass** sie aus Abschnitten (32) einer Bandage aus einem gewebten oder Vliesstoff besteht, der mehrere Abschnitte (32) umfasst, wobei die Abschnitte über wasserlösliches Fasermaterial zusammengehalten werden (34).

2. Bandage mit länglicher Form gemäß Anspruch 1, bei welcher die Abschnitte der Bandage mit wasserlöslichem Faden zusammengenäht sind.

3. Bandage mit länglicher Form gemäß Anspruch 1, bei welcher die Abschnitte der Bandage (32) durch wasserlösliches Vliesmaterial miteinander verbunden sind, welches mit jedem Abschnitt der Bandage durch Vernadelung verbunden ist.

4. Bandage mit länglicher Form gemäß Anspruch 1, bei welcher die Abschnitte der Bandage (32) durch wasserlösliches Vliesmaterial miteinander verbunden sind, welches mit jedem Abschnitt der Bandage durch Klebstoff verbunden ist.

## Revendications

1. Bandage (30) de forme allongée, **caractérisé en ce qu'**il est constitué de sections (32) de bandage d'un tissu tissé ou non tissé, comprenant de multiples sections (32), lesdites sections étant maintenues ensemble (34) par l'intermédiaire d'un matériau fibreux soluble dans l'eau.

2. Bandage de forme allongée selon la revendication 1, dans lequel les sections du bandage sont cousues ensemble avec du fil soluble dans l'eau.

3. Bandage de forme allongée selon la revendication 1, dans lequel les sections du bandage (32) sont unies ensemble par un matériau non tissé soluble dans l'eau qui est uni à chaque section du bandage par aiguilletage.

4. Bandage de forme allongée selon la revendication 1, dans lequel les sections du bandage (32) sont unies ensemble par un matériau non tissé soluble dans l'eau qui est uni à chaque section du bandage par un adhésif.
